(19) Europäisches Patentamt
European Patent Office
Office européen des brevets

(11) Publication number : **0 353 721 B1**

(12) # EUROPEAN PATENT SPECIFICATION

(45) Date of publication of patent specification : **25.11.92 Bulletin 92/48**

(51) Int. Cl.$^5$ : **C07B 39/00,** C07C 31/40, C07C 239/20, C07C 239/00

(21) Application number : **89114221.8**

(22) Date of filing : **01.08.89**

(54) **Process for preparing fluorinated compounds and products obtainable thereby.**

(30) Priority : **02.08.88 IT 2161588**

(43) Date of publication of application : **07.02.90 Bulletin 90/06**

(45) Publication of the grant of the patent : **25.11.92 Bulletin 92/48**

(84) Designated Contracting States : **DE FR GB**

(56) References cited :
**US-A- 4 010 212**
**CHEMICAL ABSTRACTS, vol. 93, no. 7, 18th August 1980, page 904, abstract no. 70905t, Columbus, Ohio, US; A. SEKIYA et al.:"Synthesis of trifluoromethyl amines,CF3N(X)H, from CF3N(X)C(O)F"**

(73) Proprietor : **AUSIMONT S.r.l.**
**Foro Buonaparte, 31**
**I-20100 Milano (IT)**

(72) Inventor : **Desmarteau, Darryl D., Dr.**
**100 Berkeley Drive**
**Clemson South Carolina 29631 (US)**
Inventor : **Kotun, Stefan P., Dr.**
**100 Berkeley Drive**
**Clemson South Carolina 29631 (US)**
Inventor : **Malacrida, Alessandro, Dr.**
**19, Via C. Battisti**
**I-20050 Sovico Milano (IT)**

(74) Representative : **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**W-8000 München 40 (DE)**

**EP 0 353 721 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for the preparation of fluorinated compounds, as well as to novel fluorinated compounds obtainable by means of said process.

US-A-4 010 212 discloses the preparation of perfluorinated tertiary alcohols from perfluoro-isoalkylene oxides-1,2 by reacting these cyclic ethers with HF in the presence of a Lewis acid.

The synthesis of trifluoromethyl amines $CF_3NHR$ (R = F, $OCF_3$, $OCF(CF_3)_2$, OMe, OEt, $OCHMe_2$, $OCMe_3$, OAc) by hydrolysis of $CF_3NRCOF$ in the presence of NaF has been reported by A. Sekiya and D. Desmarteau (Chemical Abstracts <u>93</u>, page 904).

The present invention relates to a process for the preparation of fluorinated compounds having the formula:

$$XH\text{-}Y\text{-}CZ_1Z_2\text{-}(CF_2)_n\text{-}F \qquad (I)$$

and

$$YH\text{-}X\text{-}(CF_2)_n\text{-}CZ_1Z_2F \qquad (II)$$

wherein:

X is $NR_x$, O, S, $CFR_x$, $(CR_x)_2$ or $CF_2$;
Y is $NR_x$, O, S, $CFR_x$, $(CR_x)_2$ or $CF_2$;
X is different from Y;
$R_x$ is a perhalogenated alkyl group containing from 1 to 4 carbon atoms;
n is either 0 or 1, with the proviso that when n = 0, X = $NR_x$;
$Z_1$ and $Z_2$, the same or different from each other, represent F, Cl, Br, H or $R_x$,

which process is characterized in that a fluorinated cyclic compound of formula:

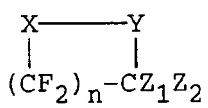

(III)

is reacted, at a temperature of from about -80°C to about +300°C, preferably from about 0°C to about 100°C, with HF in the presence of a Lewis acid.

In the process of the present invention, particularly preferred compounds of formula (III) are those wherein X (or Y) is $NR_x$, $C(R_x)_2$ $CFR_x$ and $CF_2$ and Y (or X) is 0. n preferably is 1 while preferred meanings of $Z_1$ and $Z_2$ are H, F and Cl. $R_x$ preferably is selected from $C_1$- and $C_2$-perhaloalkyl groups, particularly $CF_3$ and $C_2F_5$. When there is more than one group $R_x$, said groups $R_x$ can be the same or different from each other. Preferred halogen atoms present in $R_x$ are F and Cl, particularly F.

The Lewis acid is preferably selected from $AsF_5$, $SbF_5$ and $BF_3$.

The most preferred Lewis acid is $AsF_5$.

At the end of the reaction, the Lewis acid and any excess HF are separated from the end product, preferably by neutralization with a neutralizing agent.

As the neutralizing agent, compounds such as NaF, KF, $KHF_2$, RbF or CsF can be used, NaF being the preferred one.

The molar ratio of HF to the florinated cyclic compound (III) usually ranges from about 2:1 to about 50:1, preferably from about 4:1 to about 25:1.

The molar ratio of the Lewis acid (e.g. $AsF_5$) to the fluorinated cyclic compound of formula (III) generally is from about 0.1:1 to about 5:1 and preferably of from about 1:1 to about 2:1.

The reaction is usually complete within 30 minutes to 3 days and, more commonly, within 1 hour to 36 hours. At the end of the reaction, the reaction mixture is preferably treated with a neutralizing agent, in order to neutralize any unreacted HF and the Lewis acid.

This neutralization step can usually be accomplished at a temperature of from about -20°C to about +50°C, and preferably from about +20°C to about +25°C.

In general, an excess of neutralizing agent is employed: this excess usually is within the range of from about 2 to about 10 mol, preferably from about 3 to about 5 mol, of neutralizing agent (e.g. NaF), per each mol of the sum of the starting HF plus the Lewis acid.

This neutralization step usually is completed within 30 minutes to 30 days.

In the fluorinated cyclic compound of formula (III) used as the starting material and in the fluorinated compounds of formulae (I) and (II) obtained, $R_x$ preferably is a perfluorinated alkyl group.

According to the structure of the starting cyclic compound of formula (III) and, in particular, according to the nature of X and of Y and of the substituents $Z_1$ and $Z_2$, the product of formula (I) or the product of formula (II), or both of them, will be obtained, as will be explained in the following with reference to specific cases.

When X in the compound of formula (II) represents $CR_xF$ or $CF_2$, said product will eliminate HF to yield the unsaturated compounds of the following formulae:

$$Y=CR_x\text{-}(CF_2)_n\text{-}CZ_1Z_2F$$

or

$$Y=CF\text{-}(CF_2)_n\text{-}CZ_1Z_2F.$$

According to a preferred embodiment of the present invention, the cyclic compound used as the starting compound has the formula:

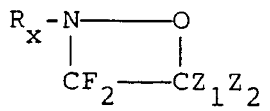

(IV)

and the end products are:
the amines of formula

$$R_x\text{-}NH\text{-}O\text{-}CZ_1Z_2\text{-}CF_3 \qquad (V)$$

and
the hydroxylamines of formula

$$R_x\text{-}NOH\text{-}CF_2\text{-}CZ_1Z_2F \qquad (VI)$$

The amines of formula (V) are new compounds. They are useful intermediates, in particular for the preparation of the corresponding N-fluorinated amines of formula:

$$R_x\text{-}NF\text{-}O\text{-}CZ_1Z_2\text{-}CF_3 \qquad (VII)$$

which are suitable as catalysts for the polymerization of $C_2F_4$. The compounds of formula (VII) are also novel and constitute a further object of the present invention.

The N-fluorinated compounds of formula (VII) can be obtained by means of the reaction of the amines of formula (V) with $F_2$ at a temperature in the range of from about -195°C to about 5°C, preferably of from about -100°C to about 0°C. The molar ratio of $F_2$ to the amine usually ranges from about 1:1 to about 2:1, preferably of from about 1.0:1 to about 1.3:1.

The preferred amines of formula (V) and N-fluorinated amines of formula (VII) are those in which $R_x$ is $CF_3$. $Z_1$ is F and $Z_2$ is F, Cl or H.

These preferred compounds are:

$$CF_3\text{-}NH\text{-}O\text{-}CF_2CF_3 \qquad (VIII)$$

N-(pentafluoroethoxy)-trifluoromethyl-amine;

$$CF_3\text{-}NH\text{-}O\text{-}CFCl\text{-}CF_3 \qquad (IX)$$

N-(1-chloro-1,2,2,2-tetrafluoroethoxy)-trifluoromethyl-amine;

$$CF_3\text{-}NH\text{-}O\text{-}CHF\text{-}CF_3 \qquad (X)$$

N-(1,2,2,2-tetrafluoro-ethoxy)-trifluoromethyl-amine;

$$CF_3\text{-}NF\text{-}OCF_2CF_3 \qquad (XI)$$

N-fluoro-N-(pentafluoroethoxy)-trifluoromethyl-amine;

$$CF_3\text{-}NF\text{-}O\text{-}CFCl\text{-}CF_3 \qquad (XII)$$

N-fluoro-N-(1-chloro-1,2,2,2-tetrafluoroethoxy)-trifluoromethyl-amine;

$$CF_3\text{-}NF\text{-}O\text{-}CFH\text{-}CF_3 \qquad (XIII)$$

N-fluoro-N-(1,2,2,2-tetrafluoroethoxy)-trifluoromethyl-amine.

The possibility of obtaining, in some cases, the amines of formula (V), and, in other cases, a mixture of amines (V) and hydroxylamines of formula (VI), when as starting material cyclic compounds of formula (IV) are used, is illustrated by the following equations (1) and (2).

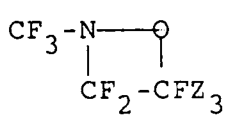

wherein $Z_3$ is F or H

$HF/AsF_5$

$$CF_3\text{-}NH\text{-}OCFZ_3\text{-}CF_3 \qquad (1)$$

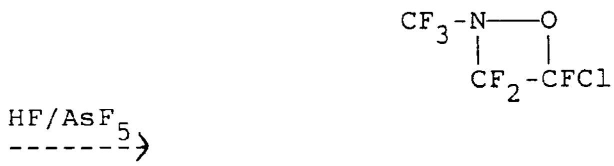


$CF_3-NH-OCFCl-CF_3$ + $CF_3-NOH-CF_2CF_2Cl$ (2)

According to another preferred embodiment of the present invention, the cyclic compound used as the starting compound is:

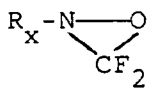
(XIV)

and the end product is

$R_x-NH-O-CF_3$ (XV)

According to a still further preferred embodiment of the present invention, the cyclic compound used as the starting compound is:

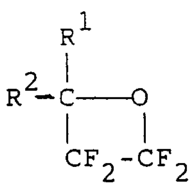
(XVI)

wherein $R^1$ and $R^2$, the same or different from each other, are F or $R_x$;
and the end product is:

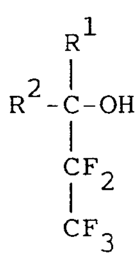
(XVII)

The following examples are given in order to illustrate the present invention without, however, being a limitation thereof.

EXAMPLE 1

This example illustrates the reaction:

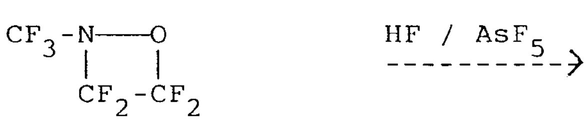


$CF_3-NH-OCF_2-CF_3$ (VIII)

(XVIII)

The perfluorinated oxazetidine (XVIII) used as the starting material was prepared from $CF_3$-N=O and $CF_2$=$CF_2$ (Barr D.A. et al, J. Chem. Soc., 1955, 1881-1889).

The reaction vessel was assembled by heat-sealing one end of a Teflon®-FEP tube of 5/8" outside diameter and 1/2" internal diameter.
The other end of the tube was inserted in a 5/8"-1/2" stainless-steel reducing unit, which, in its turn, was connected to a stainless-steel valve through a 1/2"-1/4" stainless-steel reducing port connector.

The internal volume of the reactor was 39.5 ml. An insert of stainless steel was placed inside the open end of the tubular FEP reactor as support.
The FEP reactor was evacuated 15 μm Hg; all of the subsequent manipulations under vacuum were carried out under this pressure) and weighed.

The reactor was cooled to -196°C, and anhydrous HF was condensed therein by static vacuum, through a stainless-steel manifold. The reactor was heated to 23°C, and was weighed again: the amount of HF was determined through the weight difference, and amounted to 0.97 g (48 mmol).

The reactor was cooled again with liquid nitrogen, and $AsF_5$ (10.0 mmol) was condensed therein by static vacuum, through a Pyrex®-glass manifold.
The reactor was heated again to 23°C, while being stirred in order to mix HF and $AsF_5$.

After cooling the reactor again down to -196°C, the above perfluorinated oxazetidine of formula (XVIII) (5.00 mmol) was condensed under static vacuum, through a vacuum glass line.

The mixture was heated with stirring to room temperature; a vigorous reaction started as soon as the mixture began to melt. The reaction was allowed to proceed at 23°C for 18 hours.

The reactor was then degassed by pumping off the gases which could not be condensed at -196°C; this operation facilitated the subsequent step of vacuum transfer.

After degassing at -196°C, the reaction mixture was heated to 23°C and volatile matter was condensed, via static vacuum, through a stainless-steel manifold, in a 150 ml reaction tank, equipped with a stainless-steel valve, cooled with liquid nitrogen and containing 12.22 g (291 mmol) of NaF powder (in order to remove HF and $AsF_5$) and also several stainless-steel balls having a diameter of 3/8". By heating the reactor (to about 100°C) and stirring for about 20 minutes the transfer of components to the tank could be accomplished.

After 2.5 hours at 23°C, with intermittent shaking, any volatiles contained in the reaction tank were slowly pumped into a Pyrex®"U"-trap cooled with liquid nitrogen. The subsequent fractionation of this material under dynamic vacuum, through a series of "U"-traps cooled at -70°C, -95°C and -196°C, afforded 4.84 mmol of compound of formula

$$CF_3\text{-NH-}OCF_2CF_3$$

inside the trap at -95°C (yield 96.8%, relative to oxazetidine (XVIII)).

$CF_3$-NH-$OCF_2CF_3$ showed the following spectra:

<u>IR</u> (3 torr): 3342 ( ν N-H,m), 1483 (sh,w), 1453 (s), 1399 (w), 1313 (vs), 1275 (sh,m), 1236 (vs), 1197 (vs), 1170 (vs), 1107 (vs), 1028 (w), 969 (w), 919 (m), 863 (m), 814 (w), 741 (m), 679 (w), 620 (w), 569 (w) $cm^{-1}$;
(m = medium; sh= shoulder; w = weak; s = strong, vs = very strong.)
<u>NMR</u>: $CF_3^AN(H)\text{-}OCF_2^BCF_3^C$ δ $^1H$ ($CDCl_3$) 6.6 (br q), $^{19}F$ ($CDCl_3$) A -70.6 (3F, d-t), B -95.3 (2F, q-q), C -84.9 ppm (3F, t), $J_{HA}$ = 8.6; $J_{AB}$ = 3.8; $J_{BC}$ = 1.7; $J_{HB}$ = $J_{HC}$ = $J_{AC}$ = 0 Hz.
<u>Mass spectrum</u>: major peaks, m/z [EI]: 219 (M+); 200 ($M\text{-}F^+$); 180 ($M\text{-}F\text{-}HF^+$); 130 ($M\text{-}HF\text{-}CF_3^+$); 119 ($CF_3CF_2^+$); 69 ($CF_3^+$) major peaks m/z [CI]: 200 ($M+1^+$); 200 ($M+1\text{-}HF^+$); 180 ($M+1\text{-}2HF^+$); 130 ($M\text{-}HF\text{-}CF_3^+$); 119 ($CF_3CF_2^+$).

<u>EXAMPLE 2</u>

This example illustrates the reaction:

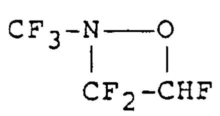

(XIX)

HF / $AsF_5$

$CF_3$-NH-OCHF-$CF_3$

(X)

Oxazetidine (XIX) was prepared by thermal cycloaddition of $CF_3$-N=O and $CF_2$=CHF (Banks R.E. et al, J. Chem. Soc., 2506-2513, 1965).
The general procedure of example 1 was followed, using a reactor having an internal volume of about 35 ml.

The following materials were introduced:

1.73 g of anhydrous HF (86 mmol);

10.00 mmol of $AsF_5$;

5.00 mmol of oxazetidine (XIX).

The mixture was heated with stirring to room temperature; a vigorous reaction started as soon as the mixture began to melt. The reaction was allowed to proceed at 23°C for 24 hours.

The neutralization was carried out with 18.16 g (432 mmol) of NaF powder.

After vacuum fractionation, 3.20 mmol of compound of formula

$CF_3$-NH-OCHF-$CF_3$

were obtained in the trap at -90°C (yield 64.0%, relative to oxazetidine).

$CF_3$-NH-OCHF-$CF_3$ showed the following spectra:

IR (3 torr): 3335 ( ν N-H,m); 2989 ( ν C-H,w); 1478 (sh,w); 1433 (s); 1409 (w); 1344 (w); 1291 (vs); 1214 (vs); 1191 (vs); 1127 (vs); 1088 (s); 1021 (w); 989 (sh,w); 923 (m); 904 (m); 830 (w); 725 (m); 700 (m); 609 ( w); 563 (w) $cm^{-1}$.

NMR: $CF_3^AN(H^B)$-$OCH^CF^DCF_3^E$ $\delta$ $^1H$ ($CDCl_3$) B 6.7 (1 H, br q); C 5.7 (1 H, d-q); $\delta$ $^{19}F$ ($CDCl_3$) A -71.2 (3F, d-d); D -144.8 (1F, d-q-q); E -82.6 ppm (3F, d-d); $J_{AB}$ = 8.4; $J_{AD}$ = 2.2; $J_{CD}$ = 57.6; $J_{CE}$ = 3.2; $J_{DE}$ = 5.9; $J_{AC}$ = $J_{AE}$ = $J_{BC}$ = $J_{BD}$ = $J_{BE}$ = 0 Hz.

Mass spectrum: major peaks m/z [EI]: 201 ($M^+$); 1.82 ($M-F^+$); 162 ($M-F-HF^+$); 132 ($M-CF_3^+$); 112 ($M-CF_3-HF^+$); 101 ($CF_3CHF^+$); 100 ($CF_3CF^+$); 69 ($CF_3^+$); major peaks m/z [CI] : 202 ($M+1^+$): 182 ($M+1-HF^+$).

EXAMPLE 3

This example illustrates the reaction:

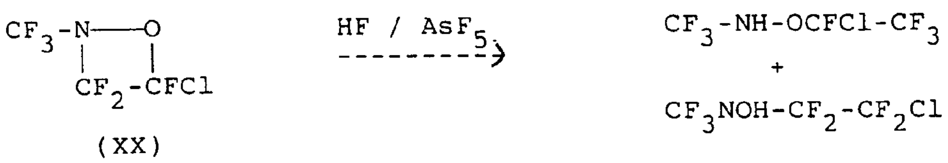


(XX) (IX)

Oxazetidine (XX) was prepared from $CF_3$-N=O nad $CF_2$=CFCl (Barr D.A. et al, J. Chem. Soc., 1961, 1351-1362).

The general procedure of example 1 was followed, using a reactor having an internal volume of 18 ml.

The following materials were introduced:

2.5 mmol of anhydrous HF;

3.7 mmol of $AsF_5$;

1.22 mmol of oxazetidine (XX).

The mixture was heated to 25°C. No reaction appeared to take place at once, but after shaking for about 5 minutes the two liquid phases, initially immiscible, became homogeneous. The mixture was left standing at 25°C for 18 hours.

The neutralization was carried out with 10.28 g (245 mmol) of NaF powder.

After the vacuum fractionation, a trap at -60°C contained 0.25 mmol of compound of formula

$CF_3$-NOH-$CF_2$-$CF_2Cl$

(yield 20%, relative to oxazetidine), and a trap at -111°C contained 0.78 mmol of

$CF_3$-NH-OCFCl-$CF_3$

(yield 64%).

$CF_3$-NH-O-CFCl-$CF_3$ showed the following spectra:

IR (4 torr): 3333 (N-H, m); 1489 (sh,w); 1450 (s); 1336 (s); 1308 (vs); 1279 (sh,m); 1228 (vs); 1206 (sh,s); 1149 (s); 1126 (s); 1093 (vs); 1022 (m); 993 (s); 918 (m), 874 (w); 839 (m); 654 (w); 563 (w) $cm^{-1}$.

NMR: $CF_3^AN(H)$-$OCF^BClCF_3^C$ $\delta$ $^1H$ ($CDCl_3$) 6.6 (br q); $\delta$ $^{19}F$ ($CDCl_3$) A -69.9 (3F, d-d); B -81.3 (1F, q-q); C -83.1 ppm (3F, d); $J_{HA}$ = 8.5; $J_{AB}$ = 4.1; $J_{BC}$ = 2.4; $J_{HB}$ = $J_{HC}$ = $J_{AC}$ = 0 Hz.

Mass spectrum: major peaks m/z [EI]: 200 ($M-Cl^+$); 180 ($M-Cl-HF^+$); 135/137 ($CFClCF_3^+$); 85/87 ($CF_2Cl^+$); 69 ($CF_3^+$); major peaks m/z [CI]: 236/238 ($M+1^+$); 216/218 ($M+1-HF^+$); 200($M-Cl^+$); 180 ($M-Cl-HF^+$); 135/137 ($CFClCF_3^+$).

EXAMPLE 4

This example illustrates the reaction:

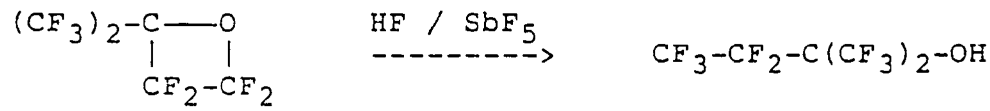


(XXI)

Oxetane (XXI) was prepared by photochemical addition of hexafluoro-acetone and $C_2F_4$ (FR-A-1,391,493).

The general procedure of example 1 was followed, using a reactor having an internal volume of 12 ml. The following materials were introduced:
2.07 g (9.55 mmol) of $SbF_5$;
2.21 g (110 mmol) of anhydrous HF;
9.55 mmol of oxetane (XXI).

The reactor was heated to 23°C whereupon the mixture still formed two liquid phases, with no indication of a reaction taking place. The reactor was then heated to 55°C for 17 hours, whereafter the mixture had become homogeneous.

The neutralization was carried out with 23.19 g (553 mmol) of NaF powder.

The vacuum fractionation yielded 9.20 mmol of compound of formula

$$CF_3CF_2C(CF_3)_2\text{-}OH$$

inside a trap at -80°C (yield 96.3%, relative to the oxetane).

EXAMPLE 5

This example illustrates the preparation of

$$CF_3\text{-}NF\text{-}OCF_2\text{-}CF_3 \qquad (XI)$$

by fluorination of $CF_3$-NH-$OCF_2CF_3$.
In all of the process steps carried out under vacuum the vacuum was 5 μm Hg.
The reactor consisted of a 150 ml stainless-steel reaction tank, equipped with a stainless-steel valve and previously passivated with 1 atm of $F_2$ for 18 hours at 23°C. The reaction tank was evacuated, cooled to -196°C, and

$$CF_3\text{-}NH\text{-}OCF_2CF_3$$

(2.50 mmol) was condensed under static vacuum through a Pyrex® glass vacuum line.
$F_2$ was introduced into the reaction tank, at -196°C, through a vacuum line of stainless steel; a 5% excess of $F_2$ was present (2.62 mmol of total $F_2$).
The liquid nitrogen used to cool the tank was removed from the Dewar bottle and the tank was allowed to slowly warm inside the empty Dewar bottle. The temperature increased from -196°C to about +5°C during 23.5 hours; thereafter the reaction tank was cooled again down to -196°C, and a trace of unreacted $F_2$ was pumped off into the stainless-steel vacuum line.

After heating the reaction tank to 23°C, volatile matter was condensed via static vacuum through the stainless-steel manifold into a 75 ml Hoke tank of stainless steel, cooled with liquid nitrogen, containing 2.01 g (47.9 mmol) of NaF powder, in order to remove HF. The NaF-containing tank was equipped with a stainless-steel valve, and the tank, already containing NaF, had previously been passivated with 1 atm of $F_2$ for 6 hours at 23°C.

The volatile matter transferred was left standing over NaF for 18 hours at 23°C. The contents of the NaF-containing tank were then carefully pumped out and collected in a "U"-shaped trap of Pyrex® glass, cooled at -196°C. The fractionation under dynamic vacuum through a series of traps at temperatures of -90°C, -135°C and -196°C yielded 2.46 mmol of compound of formula

$$CF_3\text{-}NF\text{-}OCF_2CF_3$$

inside the trap at -135°C (yield 98.4%, relative to $CF_3$ -NH-$OCF_2CF_3$ ).
$CF_3$ -NF-$OCF_2CF_3$ showed the following spectra:
IR (4 torr): 2304 (vw); 1400 (w); 1277 (vs); 1243 (vs); 1209 (sh,s); 1193 (vs); 1098 (vs); 998 (m); 932 (m); 889 (m); 826 (vw); 753 (w); 696 (m); 655 (w); 604 (vw); 531 (vw) $cm^{-1}$;
NMR: $CF_3^CN(F^D)\text{-}OCF^AF^BCF_3^E$ $^{19}F$ ($CDCl_3$, 23°C) A -93.3 (1F, m); B -95.6 (1F, m); C -81.1 (3F, br s); D +10.5 (1F, br s); E -84.9 ppm (3F, q); $J_{AB}$ = 142.1; $J_{AC}$ = 1.7; $J_{AD}$ = 5.2; $J_{AE}$ = 1.7; $J_{BD}$ = 7.4; $J_{BE}$ = 1.7; $J_{CD}$ = 1.5; $J_{DE}$

= 1.6; $J_{BC} = J_{CE} = 0$ Hz.
Mass spectrum: major peaks m/z [EI]: 119 ($C_2F_5^+$); 69 ($CF_3^+$); 50 ($CF_2^+$); major peaks m/z [CI]: 238 ($M+1^+$); 218 ($M+1-HF^+$); 130 ($CF_2NOCF_2^+$); 119 ($C_2F_5^+$).

EXAMPLE 6

This example illustrates the polymerization of $C_2F_4$, catalyzed by $CF_3$-NF-$OCF_2CF_3$.

$C_2F_4$ (PCR, Inc.) was washed, in order to remove the polymerization inhibitor (d-limonene, boiling point 178°C), by causing it to flow through a trap cooled at -111°C, under dynamic vacuum conditions. The no longer inhibited $C_2F_4$ was collected inside a trap at -196°C. This operation, as well as all subsequent operations carried out under vacuum, was conducted under a vacuum of 5 µm Hg.

Inhibitor-free $C_2F_4$ (2.00 mmol) and $CF_3$-NF-$OCF_2CF_3$ (0.20 mmol) were condensed via static vacuum inside a Pyrex®-glass tube cooled with liquid nitrogen; the tube had an internal volume of 48.8 ml and was equipped with a glass/Teflon® stopcock. The tube and its contents were allowed to warm up to 23°C, and were heated, by using a heating tape, at 150°C for 12 hours.

After cooling to room temperature and after removing the heating tape, the tube was found to contain a soft fluffy mass constituted by a white solid. The residual volatile matter was carefully pumped off and collected inside a liquid-nitrogen trap. No non-condensable gases could be detected. The PTFE remaining inside the reaction tube weighed 140 mg, by weight difference, and represented 1.40 mmol, i.e., 70% of the tetrafluoroethylene initially employed.

The fractionation of the residual volatile matter, by operating under dynamic vacuum conditions, through traps cooled at -135°C and at -196°C, yielded 0.21 mmol of material inside the trap at -135°C; the IR spectrum of said material indicated that it was constituted by the catalyst of formula

$$CF_3\text{-NF-}OCF_2CF_3$$

(quantitative recovery).

The trap at -196°C contained 0.55 mmol of $C_2F_4$, i.e., 27.5% of the olefin initially employed.

## Claims

1. Process for the preparation of fluorinated compounds of general formulae

$$XH\text{-}Y\text{-}CZ_1Z_2\text{-}(CF_2)_n\text{-}F \qquad (I)$$

and

$$YH\text{-}X\text{-}(CF_2)_n\text{-}CZ_1Z_2F \qquad (II)$$

wherein:

X and Y, which are different from each other, both may represent $NR_x$, O, S, $CFR_x$, $C(R_x)_2$ or $CF_2$;
$R_x$ is a perhalogenated alkyl group containing from 1 to 4 carbon atoms;
n is 0 or 1, with the proviso that when n = 0, X = $NR_x$;
$Z_1$ and $Z_2$, the same or different from each other, represent F, Cl, Br, H or $R_x$,

characterized in that a fluorinated cyclic compound of formula:

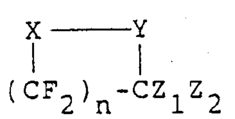

(III)

wherein X, Y, $R_x$, n, $Z_1$ and $Z_2$ are defined as above, is reacted, at a temperature of from about -80°C to about 300°C, with HF in the presence of a Lewis acid.

2. Process according to claim 1, characterised in that the Lewis acid is selected from fluorinated Lewis acids, particularly $AsF_5$, $SbF_5$ and $BF_3$.

3. Process according to any of claims 1 and 2, characterized in that at the end of the reaction, the Lewis acid and any excess HF are separated from the end product by neutralization with a neutralizing agent.

4. Process according to one or more of the preceding claims, characterized in that the reaction is carried out at a temperature of from about 0°C to about 100°C.

5. Process according to one or more of the preceding claims, characterized in that $R_x$ is a perfluorinated alkyl group.

6. Process according to one or more of the preceding claims, characterized in that the fluorinated cyclic compound has the formula:

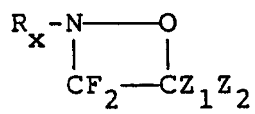

(IV)

and the end products have the formulae:

$R_x$-NH-O-$CZ_1Z_2$-$CF_3$ (V)

and

$R_x$-NOH-$CF_2$-$CZ_1Z_2F$; (VI)

or the fluorinated cyclic compound has the formula:

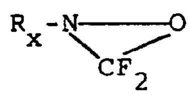

(XIV)

and the end product has the formula:

$R_x$-NH-O-$CF_3$; (XV)

or the fluorinated cyclic compound has the formula:

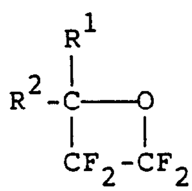

(XVI)

wherein $R^1$ and $R^2$, the same or different from each other, are F or $R_x$, and the end product has the formula:

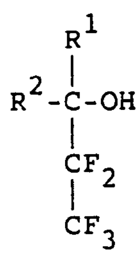

(XVII)

$R_x$, $Z_1$ and $Z_2$ being defined as in any one of claims 1 and 5.

7. N-Perhaloalkyl-alkoxyamines having the general formula:

$R_x$-NH-O-$CZ_1Z_2$-$CF_3$ (V)

wherein:

$R_x$ is a perhalogenated alkyl group containing from 1 to 4 carbon atoms;
$Z_1$ and $Z_2$, the same or different from each other, represent F, Cl, Br or H.

8. Compounds according to claim 7, wherein $R_x$ is a perfluorinated alkyl group, and particularly compounds of the following formulae (VIII) to (X):

$CF_3$-NH-O-$CF_2CF_3$ (VIII)
$CF_3$-NH-O-$CFClCF_3$ (IX)
$CF_3$-NH-O-$CHFCF_3$ (X)

9. N-Fluoro-N-perhaloalkyl-alkoxy-amines of general formula:

$R_x$-NF-O-$CZ_1Z_2$-$CF_3$ (VII)

wherein:

$R_x$ is a perhalogenated alkyl group, containing from 1 to 4 carbon atoms;

$Z_1$ and $Z_2$, the same or different from each other, represent F, Cl, Br, H or $R_x$.

**10.** Compounds according to claim 9, wherein $R_x$ is a perfluorinated alkyl group, and particularly compounds of the following formulae (XI) to (XIII):

$CF_3$-NF-O-$CF_2CF_3$ (XI)

$CF_3$-NF-O-$CFClCF_3$ (XII)

$CF_3$-NF-O-$CHFCF_3$ (XIII)

## Patentansprüche

**1.** Verfahren zur Herstellung von fluorierten Verbindungen der allgemeinen Formeln

XH-Y-$CZ_1Z_2$-$(CF_2)_n$-F (I)

und

YH-X-$(CF_2)_n$-$CZ_1Z_2F$ (II)

worin:

X und Y, die verschieden voneinander sind, beide $NR_x$, O, s, $CFR_x$, $C(R_x)_2$ oder $CF_2$ repräsentieren können;

$R_x$ eine perhalogenierte Alkylgruppe mit 1 bis 4 Kohlenstoffatomen ist;

n 0 oder 1 ist, mit der Maßgabe, daß wenn n = 0, X = $NR_x$;

$Z_1$ und $Z_2$,gleich oder verschieden voneinander, F, Cl, Br, H oder $R_x$ repräsentieren,

dadurch gekennzeichnet, daß eine fluorierte cyclische Verbindung der Formel

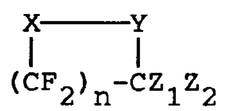

(III)

worin X, Y, $R_x$, n, $Z_1$ und $Z_2$ wie oben definiert sind, bei einer Temperatur von etwa -80°C bis etwa 300°C mit HF in Gegenwart einer Lewis-Säure umgesetzt wird.

**2.** Verfahren nach Anspruch 1, dadurch gekennzeichnet, daß die Lewis-Säure aus fluorierten Lewis-Säuren, insbesondere $AsF_5$, $SbF_5$ und $BF_3$ ausgewählt ist.

**3.** Verfahren nach irgendeinem der Ansprüche 1 oder 2, dadurch gekennzeichnet, daß am Ende der Reaktion die Lewis-Säure und jeglicher HF-Überschuff durch Neutralisation mit einem Neutralisierungsmittel vom Endprodukt abgetrennt werden.

**4.** Verfahren nach einem oder mehreren der vorhergehenden Ansprüche, dadurch gekennzeichnet, daß die Reaktion bei einer Temperatur von etwa O°C bis etwa 100°C durchgeführt wird.

**5.** Verfahren nach einem oder mehreren der vorhergehenden Ansprüche, dadurch gekennzeichnet, daß $R_x$ eine perfluorierte Alkylgruppe ist.

**6.** Verfahren nach einem oder mehreren der vorhergehenden Ansprüche, dadurch gekennzeichnet, daß die fluorierte cyclische Verbindung die Formel

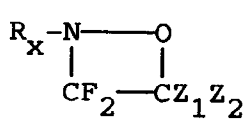

(IV)

aufweist und die Endprodukte die Formeln

$R_x$-NH-O-$CZ_1Z_2$-$CF_3$ (V)

und

$R_x$-NOH-$CF_2$-$CZ_1Z_2F$ (VI)

aufweisen; oder die fluorierte cyclische Verbindung die Formel

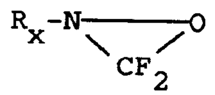

(XIV)

aufweist und das Endprodukt die Formel

$R_x$-NH-O-$CF_3$ (XV)

aufweist; oder die fluorierte cyclische Verbindung die Formel

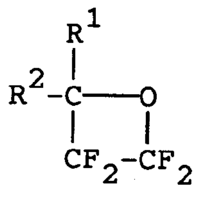

(XVI)

aufweist, worin $R^1$ und $R^2$, gleich oder verschieden voneinander, F oder $R_x$ sind und das Endprodukt die Formel

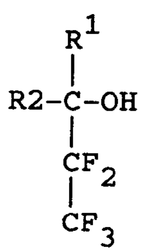

(XVII)

aufweist, wobei $R_x$, $Z_1$ und $Z_2$ wie in irgendeinem der Ansprüche 1 oder 5 definiert sind.

**7.** N-Perhalogenalkylalkoxyamine der allgemeinen Formel

$R_x$-NH-O-$CZ_1Z_2$-$CF_3$ (V)

worin:

$R_x$ eine perhalogenierte Alkylgruppe mit 1 bis 4 Kohlenstoffatomen ist;
$Z_1$ und $Z_2$,gleich oder verschieden voneinander, F, Cl, Br oder H repräsentieren.

**8.** Verbindungen nach Anspruch 7, worin $R_x$ eine perfluorierte Alkylgruppe ist, und insbesondere Verbindungen der folgenden Formeln (VIII) bis (X):

$CF_3$-NH-O-$CF_2CF_3$ (VIII)
$CF_3$-NH-O-$CFClCF_3$ (IX)
$CF_3$-NH-O-$CHFCF_3$ (X)

**9.** N-Fluor-N-perhalogenalkylalkoxyamine der allgemeinen Formel

$R_x$-NF-O-$CZ_1Z_2$-$CF_3$ (VII)

worin:

$R_x$ eine perhalogenierte Alkylgruppe mit 1 bis 4 Kohlenstoffatomen ist;
$Z_1$ und $Z_2$, gleich oder verschieden voneinander, F, Cl, Br, H oder $R_x$ repräsentieren.

**10.** Verbindungen nach Anspruch 9, worin $R_x$ eine perfluorierte Alkylgruppe ist, und insbesondere Verbindungen der folgenden Formeln (XI) bis (XIII):

$CF_3$-NF-O-$CF_2CF_3$ (XI)
$CF_3$-NF-O-$CFClCF_3$ (XII)
$CF_3$-NF-O-$CHFCF_3$ (XIII).

## Revendications

1. Procédé pour la préparation de composés fluorés ayant les formules générales:

$$XH\text{-}Y\text{-}CZ_1Z_2\text{-}(CF_2)_n\text{-}F \qquad (I)$$

et

$$YH\text{-}X\text{-}(CF_2)_n\text{-}CZ_1Z_2F \qquad (II)$$

dans lesquelles:

X et Y, qui sont différents l'un de l'autre, peuvent tous les deux représenter $NR_x$, O, S, $CFR_x$, $(CR_x)_2$ ou $CF_2$;

$R_x$ est un groupe alkyle perhalogéné contenant 1 à 4 atomes de carbone;

n est zéro ou un, à condition que lorsque n = O, X = $NR_x$;

$Z_1$ et $Z_2$ sont identiques ou différents et représentent F, Cl, Br, H ou $R_x$.

caractérisé en ce qu'un composé cyclique fluoré de formule:

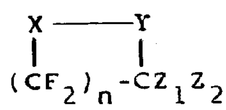

(III)

dans laquelle X, Y, $R_x$, n, $Z_1$ et $Z_2$ sont tels que définis plus haut,
est traité, à une température d'environ -80°C à environ +300°C, avec du HF en présence d'un acide de Lewis.

2. Procédé suivant la revendication 1, caractérisé en ce que l'acide de Lewis est choisi parmi les acides de Lewis fluorés, en particulier $AsF_5$, $SbF_5$ et $BF_3$.

3. Procédé suivant l'une quelconque des revendications 1 et 2, caractérisé en ce qu'à la fin de la réaction, l'acide de Lewis et l'éventuel excès de HF sont séparés du produit final par neutralisation avec un agent neutralisant.

4. Procédé suivant l'une quelconque des revendications précédentes, caractérisé en ce que la réaction est effectuée à une température d'environ 0°C à environ 100°C.

5. Procédé suivant l'une quelconque des revendications précédentes, caractérisé en ce que $R_x$ est un groupe alkyle perfluoré.

6. Procédé suivant l'une quelconque des revendications précédentes, caractérisé en ce que le composé cyclique fluoré a la formule:

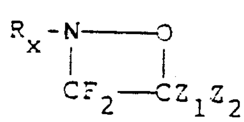

(IV)

et les produits finaux ont les formules:

$$R_x\text{-}NH\text{-}O\text{-}CZ_1Z_2\text{-}CF_3 \qquad (V)$$

et

$$R_x\text{-}NOH\text{-}CF_2\text{-}CZ_1Z_2F \qquad (VI)$$

ou que le composé cyclique fluoré a la formule:

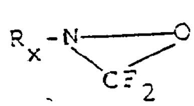

(XIV)

et le produit final a la formule suivante:

$R_x$-NH-O-$CF_3$ (XV)

ou que le composé cyclique fluoré a la formule:

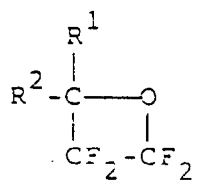

(XVI)

dans laquelle $R^1$ et $R^2$ sont identiques ou différents et représentent F ou $R_x$, et le produit final a la formule:

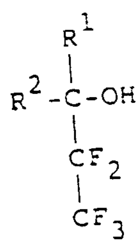

(XVII)

dans laquelle $R_x$, $Z_1$ et $Z_2$ sont tels que définis dans l'une quelconque des revendications 1 et 5.

7. N-Perhaloalkyl-alcoxyamines ayant la formule générale:

$R_x$-NH-O-$CZ_1Z_2$-$CF_3$ (V)

dans laquelle:

$R_x$ est un groupe alkyle perhalogéné contenant de 1 à 4 atomes de carbone;

$Z_1$ et $Z_2$, identiques ou différents l'un de l'autre, sont F, Cl, Br ou H.

8. Composés suivant la revendication 7, dans laquelle $R_x$ est un groupe alkyle perfluoré et en particulier, composés ayant les formules suivantes (VIII) à (X):

$CF_3$-NH-O-$CF_2$-$CF_3$ (VIII)

$CF_3$-NH-O-CFCl-$CF_3$ (IX)

$CF_3$-NH-O-CHF-$CF_3$ (X)

9. N-Fluoro-N-perhaloalkyl-alcoxyamines ayant la formule générale:

$R_x$-NF-O-$CZ_1Z_2$-$CF_3$ (VII)

dans laquelle:

$R_x$ est un groupe alkyle perhalogéné contenant de 1 à 4 atomes de carbone;

$Z_1$ et $Z_2$, identiques ou différents l'un de l'autre, sont F, Cl, Br, H ou $R_x$.

10. Composés suivant la revendication 9, dans laquelle $R_x$ est un groupe alkyle perfluoré et en particulier, composés ayant les formules suivantes (XI) à (XIII):

$CF_3$-NF-O-$CF_2$-$CF_3$ (XI)

$CF_3$-NF-O-CFCl-$CF_3$ (XII)

$CF_3$-NF-O-CHF-$CF_3$ (XIII)